# EUROPÄISCHE PATENTSCHRIFT

(11) **EP 2 313 028 B1**
(45) Veröffentlichungstag und Bekanntmachung des Hinweises auf die Patenterteilung: **19.07.2017**
(21) Anmeldenummer: 09777807.0
(22) Anmeldetag: 11.08.2009
(51) Int. Cl.: A61F 2/30, A61F 2/08, B22D 17/00

(54) **INTERFERENZSCHRAUBE**
INTERFERNCE SCREW
VIS D'INTERFERENCE

(30) Priorität: 11.08.2008 DE 102008037202
(43) Veröffentlichungstag der Anmeldung: 27.04.2011
(73) Patentinhaber: aap Implantate AG, 12099 Berlin (DE)
(72) Erfinder: FISCHER, Hans-Joachim, 12277 Berlin (DE); WOLFSTÄDTER, Marco, 63939 Wörth/Main (DE); WITTE, Frank, 30171 Hannover (DE); HORT, Norbert, 21339 Lüneburg (DE); FRÖHLICH, Bernd, 34225 Baunatal (DE); VOITH, Wolfgang, 78554 Aldingen (DE)
(74) Vertreter: Herden, Andreas F.
(86) Internationale Anmeldenummer: PCT/EP2009/005819
(87) Internationale Veröffentlichungsnummer: WO 2010/017960

(56) Entgegenhaltungen:
- EP-A1- 1 093 773
- EP-A1- 1 378 205
- WO-A1-02/100452
- WO-A2-2008/021474
- DE-U1-202006 009 843
- US-A- 4 927 421
- US-A1- 2002 055 783
- US-B1- 6 629 977
- US-B2- 7 037 309

## Beschreibung

### Gebiet der Erfindung

Die Erfindung betrifft eine Interferenzschraube, insbesondere eine Interferenzschraube zur endostalen Fixierung eines Ligamentes, insbesondere eines Ersatzligamentes eines Kreuzbandes.

### Hintergrund der Erfindung

Interferenzschrauben für medizinische Anwendungen sind bekannt. Es handelt sich dabei um Schrauben, welche in eine zuvor gesetzte Bohrung oder einen Markkanal eingedreht werden und nach dem Eindrehen durch eine Übermaßpassung in dem Kanal fixiert sind.

Eine derartige gattungsbildende Interferenzschraube zeigt beispielsweise das deutsche Gebrauchsmuster DE 9320384.

Bei bekannten Interferenzschrauben hat sich gezeigt, dass diese sich unter ungünstigen Bedingungen dadurch lockern können, dass sie sich aus dem Kanal herausdrehen.

Weiter können bekannte Interferenzschrauben aus Metall in der Regel nur mit aufwendigen spanabhebenden Verfahren hergestellt werden.

Das Dokument WO 2008/021474 A2 zeigt eine Schraube mit Öffnungen im Gewindegang, über die ein besserer Kontakt zum Gewebe hergestellt werden soll. Die Dokumente US 4,927,421 A, US 7,037,309 und EP 1 378 205 A1 zeigen selbstschneidende Schrauben, welche jeweils in das Gewinde eingebrachte Schnittflächen umfassen.

### Aufgabe der Erfindung

Der Erfindung liegt demgegenüber die Aufgabe zugrunde, eine Interferenzschraube bereitzustellen, welche weniger dazu neigt, sich zu lockern.

Weiter ist es insbesondere eine Aufgabe der Erfindung, eine Schraube bereitzustellen, welche mit möglichst geringem Widerstand eindrehbar ist.

Eine weitere Aufgabe der Erfindung besteht vorzugsweise darin, eine bioresorbierbare Schraube aus einer Metalllegierung im Druckgussverfahren herstellen zu können, wobei die Schraube keine scharfen Kanten hat, die bei Verwendung der Schrauben zu Einschnitten des Gewebes führen können.

### Zusammenfassung der Erfindung

Die Aufgabe der Erfindung wird bereits durch eine Interferenzschraube nach Anspruch 1 gelöst.

Bevorzugte Ausführungsformen und Weiterbildungen der Erfindung sind den jeweiligen Unteransprüchen zu entnehmen.

Die Erfindung betrifft eine Interferenzschraube mit einem Außengewinde, also eine Schraube, welche aufgrund eines Übermaßes in einem Bohrloch oder Knochenkanal festsitzt. Insbesondere ist die Interferenzschraube zur Fixierung eines Ligamentes, insbesondere eines Kreuzbandligamentes ausgebildet.

Gemäß der Erfindung weisen die Gewindegänge des Außengewindes zumindest abschnittsweise Ausnehmungen auf.

Die Erfinder haben herausgefunden, dass Ausnehmungen, welche vorzugsweise als Abflachung in den Gewindegängen ausgebildet sind, als Verdrehsicherung wirksam sind.

Vorzugsweise sind die Ausnehmungen in axialer Richtung hintereinander angeordnet.

Die Ausnehmungen sind als Abflachungen zumindest des Zahngrundes ausgebildet, wohingegen die Zahnköpfe vorzugsweise nicht mit Abflachungen versehen sind. Unter eine Abflachung wird jeglicher Materialabtrag im Zahngrund über einen bestimmten Winkelbereich gegenüber dem verbleibenden Umfang des Gewindes verstanden.

Vorzugsweise weist zumindest die Hälfte der eine Ausnehmung auf. So ist eine gute Wirkung als Verdrehsicherung sicher gestellt.

Sofern man das Gewinde beidseitig mit einer Ausnehmung versieht, lässt sich eine derartige Schraube besonders gut in einem Druckgussverfahren herstellen, bei welchem die Form entlang der Ausnehmungen geöffnet wird. Es hat sich herausgestellt, dass sich durch die Ausnehmungen eine Gradbildung fast vollständig vermeiden lässt.

Gemäß der Erfindung bilden die Ausnehmungen zumindest abschnittsweise eine Sekantenfläche zur Zahnflanke und zum Zahngrund des Gewindes. Die Sekantenfläche verläuft entlang einer in die Zahnflanken und den Zahngrund hineinragenden Ausnehmung und streicht vor Erreichen des Zahnkopfes des Gewindes an den Zahnflanken heraus. Die Zahnköpfe des Gewindes werden somit nicht von der Ausnehmung erfasst. Vielmehr wird bei einer bevorzugten Ausführungsform der Erfindung die Ausnehmung allein durch eine Abflachung der Gewindegänge gebildet.

Die Sekantenfläche verläuft dabei vorzugsweise entlang tangential zur Schraube verlaufender Linien, ist also gerade, so dass eine Tangente eines Kreises, dessen Mittelpunkt auf der Mittelachse der Interferenzschraube liegt, im Wesentlichen auf der Oberfläche der Ausnehmung liegt. Die so in einer tangentialen Richtung gerade ausgebildete Sekantenfläche ermöglicht eine leichtere Entformung der Schraube in einem Druckgussverfahren.

Bei einer bevorzugten Ausführungsform der Erfindung dringt die Ausnehmung an der tiefsten Stelle am Zahngrund des Gewindes zwischen 0,05 und 1 mm in den Schraubenkörper ein, während sie sich auf den Flanken des Gewindes in Richtung des Zahnkopfes verjüngt und noch auf den Flanken der Zähne aus dem Schraubenkörper ausstreicht.

In axialer Vorderansicht nimmt die Ausnehmung nur einen Teilbereich des Schraubenkörpers ein, vorzugsweise nimmt die Ausnehmung einen Winkel von weniger als 50°, besonders bevorzugt von weniger als 40°, im Gewindegang ein.
Unter diesem Winkel wird der Winkel verstanden, an welchem die am weitest entfernten Punkte einer Ausnehmung in einem Gewindegang auseinander liegen. In axialer Richtung ist vorzugsweise im Wesentlichen das gesamte Gewinde mit Ausnehmungen versehen. Lediglich an einem sich verjüngenden Schraubenkopf sind bei einer weiteren Ausführungsform der Erfindung keine Ausnehmungen vorgesehen.

Bei einer Weiterbildung der Erfindung weist die Interferenzschraube einen Innenkanal auf, vorzugsweise um einen Führungsdraht oder Führungsstift aufzunehmen.

Bei einer weiteren Ausführungsform der Erfindung ist die Interferenzschraube aus einem bioresorbierbaren Material ausgebildet. Die Erfinder haben herausgefunden, dass sich hierfür insbesondere eine Magnesium-Druckgusslegierung eignet.

Das Gewinde der Schraube hat bei einer bevorzugten Ausführungsform der Erfindung eine Steigung zwischen 2 und 3 mm. Diese Steigung hat sich insbesondere zum Eindrehen in Knochenmaterial als besonders geeignet erwiesen.

Weiter hat sich herausgestellt, dass besonders ein Sägezahnprofil ein leichtes Eindrehen der Schraube und dabei dennoch eine gute Fixierbarkeit ermöglicht.
Dabei stehen vorzugsweise die Flanken auf der Vorderseite des Gewindes, also der der Eindrehrichtung zugewandten Flanke, in einem Winkel zwischen 25° und 35° zu einer senkrecht zur Mittelachse der Schraube verlaufenden Ebene, wohingegen die Flanken auf der Rückseite in einem kleineren Winkel zu einer senkrecht zur Mittelachse der Schraube verlaufenden Ebene von 10° bis 20° stehen.

Um insbesondere Beschädigungen des Ligamentes durch Kanten zu verringern, sind bei einer Ausführungsform der Erfindung die Zahnköpfe des Gewindes abgerundet.

Zum Eindrehen in Knochenmaterial hat sich ein Verhältnis des Flankendurchmessers zum Kerndurchmesser von 1,4 bis 1,8, besonders bevorzugt von 1,5 bis 1,6 als besonders geeignet gezeigt.

### Kurzbeschreibung der Zeichnungen

Die Erfindung soll im Folgenden bezugnehmend auf die Zeichnungen Fig. 1 bis Fig. 6 näher erläutert werden.
Fig. 1 zeigt eine dreidimensionale Seitenansicht eines schematisch dargestellten Ausführungsbeispiels einer Interferenzschraube,
Fig. 2 zeigt eine Schnittansicht entlang der Mittelachse,
Fig. 3 zeigt eine axiale Vorderansicht auf die Rückseite der Schraube,
Fig. 4 zeigt eine Detailansicht der Ausnehmung,
Fig. 5 und 6 zeigen schematisch Herstellungsschritte eines Druckgussverfahrens für eine Interferenzschraube.

### Detaillierte Beschreibung der Zeichnungen

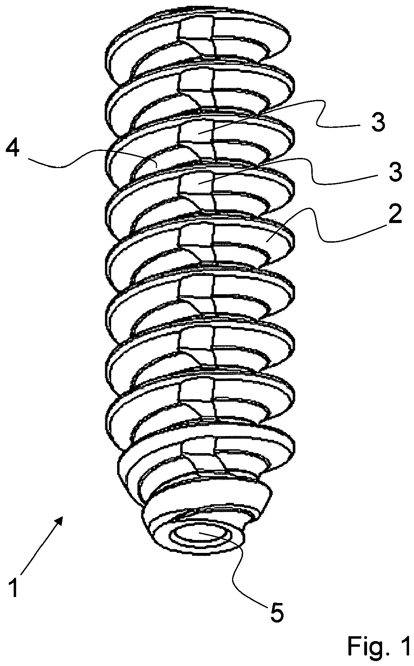

Fig. 1 zeigt eine dreidimensional dargestellte Seitenansicht einer Interferenzschraube.

Die Interferenzschraube 1 umfasst einen Schraubenkörper mit einem im Wesentlichen sägezahnförmig ausgestalteten Gewinde 2. An der Spitze verjüngt sich das Gewinde. Weiter weist der Schraubenkörper einen Kanal 5 auf, mittels dessen die Interferenzschraube auf einem Draht oder einem Dorn zentriert werden kann.

Die Gewindegänge 4 der Interferenzschraube weisen Ausnehmungen 3 auf, welche sich im Wesentlichen über die gesamte Länge der Schraube erstrecken.

Die Ausnehmungen 3 sind jeweils als Sekantenflächen ausgebildet, welche an den Flanken der Zähne der Schraube in den Schraubenkörper einstreichen, im Zahngrund, den im Wesentlichen tiefsten Punkt erreichen und auf der gegenüberliegenden Seite wieder aus der Flanke herausstreichen.

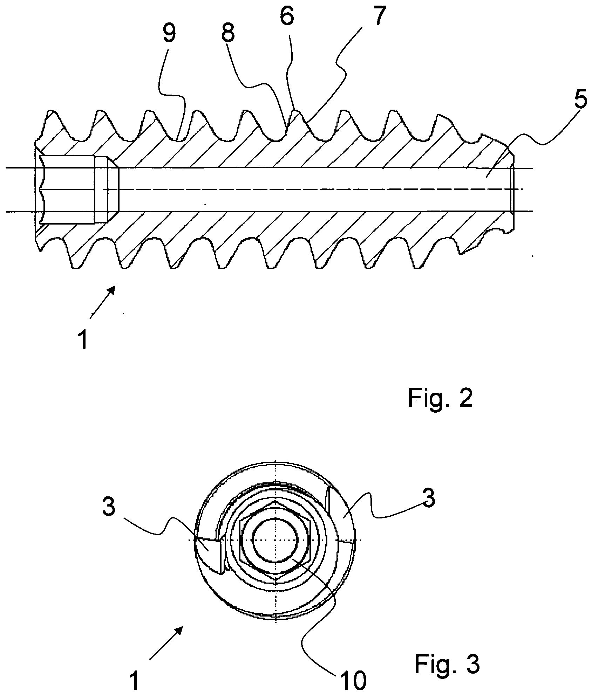

Fig. 2 zeigt eine Schnittansicht der in Fig. 1 dargestellten Interferenzschraube 1. Zu erkennen ist der Kanal 5, welcher sich über die gesamte Länge der Schraube erstreckt. Anhand dieser Zeichnung soll in erster Linie die Definition der verschiedenen Bereiche des Gewindes erläutert werden.

Das Gewinde besteht aus Zähnen, die eine vordere Flanke 7, eine hintere Flanke 8 sowie einen Zahnkopf 6 und einen Zahngrund 9 aufweisen.

Bei einer bevorzugten Ausführungsform der Erfindung streicht die Ausnehmung (in dieser Ansicht nicht dargestellt), im Wesentlichen kurz vor Erreichen des Zahnkopfes 6 aus dem Schraubenkörper aus.

Dies ist auch in Fig. 3 gut zu erkennen, in der eine Vorderansicht auf die Rückseite der Interferenzschraube 1 dargestellt ist. Hier ist zu erkennen, dass sich der Flankendurchmesser der Interferenzschraube 1 auch im Bereich der Ausnehmungen 3 im Wesentlichen nicht verjüngt. Als Antrieb für ein Handhabungswerkzeug weist die Schraube auf der Rückseite beispielsweise einen Sechskant 10 oder einen Torxantrieb (nicht dargestellt) auf. Durch die Ausnehmungen 3 konnte erreicht werden, dass bei einer im Druckgussverfahren aus einer Magnesiumlegierung hergestellten Interferenzschraube 1 so gut wie keine Überstände vorhanden sind. Des Weiteren dienen die Ausnehmungen 3 zugleich als Verdrehsicherung.

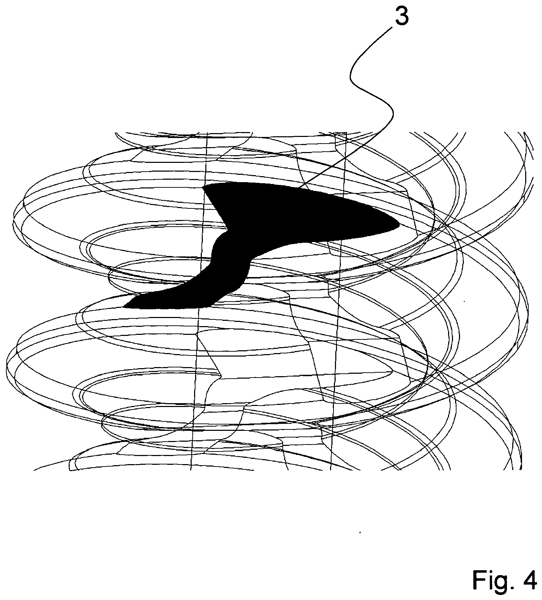

Fig. 4 zeigt eine Detailansicht einer Interferenzschraube im Bereich einer Ausnehmung 3, welche als schwarze Fläche dargestellt ist.

Die als Sekantenfläche ausgebildete Ausnehmung steht tangential zu einem Kreis, der seinen Mittelpunkt in der Achse der Schraube hat. Gleichzeitig verläuft die als Sekantenfläche ausgebildete Ausnehmung 3 entlang einer Leitkurve, die an einer Flanke in den Schraubenkörper eintaucht, am Zahngrund die tiefste Stelle erreicht und auf der gegenüberliegenden Seite aus dem Schraubenkörper herausstreicht.

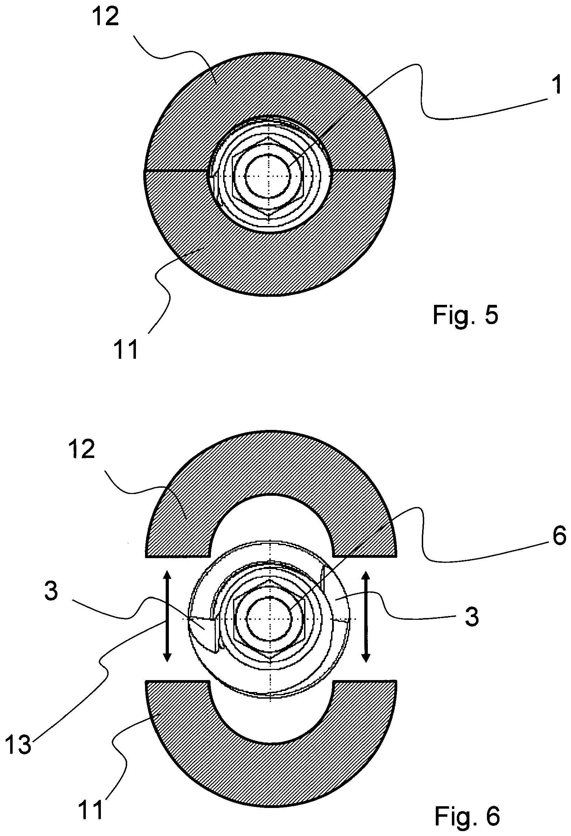

Bezug nehmend auf Fig. 5 und 6 soll eine Möglichkeit der Herstellung einer Interferenzschraube näher erläutert werden.

Die Interferenzschraube 1 wird mittels eines Druckgussverfahrens hergestellt. Dabei wird die zumindest noch zähflüssige Schmelze in eine Form gepresst, welche ein Oberteil 12 und ein Unterteil 11 aufweist, hier schematisch in einer Schnittansicht gezeigt. Sobald das Metall die Wände der Form berührt, bildet sich eine Haut, die zu der vorteilhaften geschlossenen Oberfläche führt.

Bei geeigneter Steuerung der Verfahrensparameter des Druckgussverfahrens, z.B. durch Einleiten der Schmelze mit hohem Druck, lässt sich im Inneren der Interferenzschraube eine poröse Struktur bereit stellen. Man vermutet, dass diese poröse Struktur durch Gaseinschlüsse oder durch Schrumpfung des Metalls verursacht wird, während sich das Material innerhalb von Millisekunden verfestigt. Es hat sich herausgestellt, dass eine derart poröse Struktur, die normalerweise beim Druckgießen gerade nicht erwünscht ist, die beschriebenen vorteilhaften Eigenschaften mit sich bringt.

Anschließend wird, wie in Fig. 6 dargestellt, die Form geöffnet. Die Öffnungsrichtung ist durch den Pfeil 13 dargestellt. Die Ausnehmungen 3 der Interferenzschraube 1 verlaufen vorzugsweise nicht genau parallel zur Öffnungsrichtung, sondern in einem kleinen Winkel von 0,1 bis 3°. Das Verfahren ermöglicht eine schnelle Herstellung einer Interferenzschraube mit wesentlich verbesserten Eigenschaften, insbesondere bezüglich des Abbaus der Schraube im Gewebe.

Es versteht sich, dass die Erfindung nicht auf eine Kombination vorstehend beschriebener Merkmale beschränkt ist, sondern dass der Fachmann sämtliche Merkmale, soweit dies sinnvoll ist, beliebig kombinieren wird.

### Bezugszeichenliste

- 1: Interferenzschraube
- 2: Gewinde
- 3: Ausnehmung
- 4: Gewindegang
- 5: Kanal
- 6: Zahnkopf
- 7: vordere Flankenfläche
- 8: hintere Flankenfläche
- 9: Zahngrund
- 10: Sechskant
- 11: Unterteil
- 12: Oberteil
- 13: Pfeil (Öffnungsrichtung)

## Patentansprüche

1. Interferenzschraube (1) mit einem Außengewinde (2), insbesondere ausgebildet zur Fixierung eines Ligamentes, wobei die Gewindegänge des Außengewindes zumindest abschnittsweise Ausnehmungen (3) aufweisen,
**dadurch gekennzeichnet, dass** die Ausnehmungen (3) als Abflachungen zumindest des Zahngrundes ausgebildet sind und zumindest abschnittsweise eine Sekantenfläche zu zumindest einer Zahnflanke und zum Zahngrund des Gewindes (2) bilden, wobei die Sekantenfläche vor Erreichen des Zahnkopfes (6) des Gewindes (2) aus den Zahnflanken herausstreicht.

2. Interferenzschraube nach Anspruch 1, **dadurch gekennzeichnet, dass** die Ausnehmungen (3) in axialer Richtung im Wesentlichen hintereinander angeordnet sind.

3. Interferenzschraube nach einem der vorstehenden Ansprüche, **dadurch gekennzeichnet, dass** zumindest die Hälfte der, vorzugsweise alle Gewindegänge (4) eine Ausnehmung aufweisen.

4. Interferenzschraube nach einem der vorstehenden Ansprüche, **dadurch gekennzeichnet, dass** eine Tangente eines Kreises, dessen Mittelpunkt auf der Mittelachse der Interferenzschraube (1) liegt, im Wesentlichen auf der Ausnehmung (3) liegt und/oder dass die Ausnehmung (3) an der tiefsten Stelle am Zahngrund (9) des Gewindes (2) zwischen 0,05 und 1 mm in den Schraubenkörper eindringt.

5. Interferenzschraube nach einem der vorstehenden Ansprüche, **dadurch gekennzeichnet, dass** sich die Ausnehmungen (3) über einen Winkel von weniger als 50°, vorzugsweise weniger als 40° erstrecken.

6. Interferenzschraube nach einem der vorstehenden Ansprüche, **dadurch gekennzeichnet, dass** sich die Ausnehmungen (3) in axialer Richtung im Wesentlichen entlang des gesamten Gewindes (2) erstrecken und/oder dass sich die Ausnehmungen (3) nicht über die Zahnköpfe (6) des Gewindes (2) erstrecken.

7. Interferenzschraube nach einem der vorstehenden Ansprüche, **dadurch gekennzeichnet, dass** die Vorderseite der Flanken des Gewindes zumindest abschnittsweise in einem größeren Winkel zu einer senkrecht zur Mittelachse der Schraube verlaufenden Ebene steht als die Rückseite der Flanken und/oder dass die Vorderseite der Flanken des Gewindes zumindest abschnittsweise in einem Winkel zwischen 25° und 35° zu einer senkrecht zur Mittelachse der Schraube verlaufenden Ebene steht und/oder dass die Rückseite der Flanken des Gewindes zumindest abschnittsweise in einem Winkel zwischen 10° und 20° zu einer senkrecht zur Mittelachse der Schraube (1) verlaufenden Ebene steht.

8. Interferenzschraube nach einem der vorstehenden Ansprüche, **dadurch gekennzeichnet, dass** die Interferenzschraube (1) zumindest abschnittsweise porös ist, insbesondere dass die Porosität von außen nach innen zunimmt.

9. Interferenzschraube nach einem der vorstehenden Ansprüche, **dadurch gekennzeichnet, dass** der Porositätsgrad zumindest abschnittsweise mehr als 5 %, bevorzugt mehr als 10 % und besonders bevorzugt mehr als 20 % beträgt und/oder dass der Porositätsgrad in einem oberflächenfernen Bereich der Schraube gegenüber einem oberflächennahen Bereich der Schraube um mindestens das 1,5-fache, vorzugsweise mindestens das 2-fache und besonders bevorzugt mindestens das 3-fache höher ist.

10. Interferenzschraube nach einem der vorstehenden Ansprüche, **dadurch gekennzeichnet, dass** die Interferenzschraube (1) eine Beschichtung aufweist, welche sich nach dem Einsetzen in einer vorbestimmten Zeitspanne auflöst oder welche nach dem Einsetzen durch eine induktive Erwärmung der Interferenzschraube entfernbar ist.

11. Verfahren zur Herstellung einer Interferenzschraube nach einem der vorstehenden Ansprüche, **dadurch gekennzeichnet, dass** die Interferenzschraube (1) mit einem Druckgussverfahren hergestellt wird, bei welchem die Druckgussform entlang abschnittsweiser Ausnehmungen (3) der Gewindegänge (4) des Außengewindes (2) geöffnet wird.

12. Verfahren zur Herstellung einer Interferenzschraube nach dem vorstehenden Anspruch, **dadurch gekennzeichnet, dass** die Ausnehmungen (3) zumindest abschnittsweise einen Winkel zwischen 0,1 und 15°, bevorzugt zwischen 0,3 und 0,7° zur Öffnungsrichtung der Druckgussform stehen.

## Claims

1. An interference screw (1) having an external thread (2), in particular adapted to fix a ligament, wherein the thread turns of the external thread have recesses (3), at least in portions thereof;
**characterized in that** the recesses (3) are formed as flattened areas in at least the tooth root, and that at least portions thereof define a secant surface with respect to at least one tooth flank and with respect to the tooth root of the thread (2), wherein the secant surface emerges from the tooth flanks before reaching the tooth crest (6) of the thread (2).

2. The interference screw according to claim 1,
**characterized in that** the recesses (3) are arranged substantially in a row in axial direction.

3. The interference screw according to any one of the preceding claims, **characterized in that** at least half of and preferably all of the thread turns (4) have a recess.

4. The interference screw according to any one of the preceding claims, **characterized in that** a tangent of a circle with a center located at the central axis of the interference screw (1) substantially extends along the recess (3), and/or that the recess (3) is recessed into the screw body between 0.05 and 1 mm at the lowest point at the tooth root (9) of the thread (2).

5. The interference screw according to any one of the preceding claims, **characterized in that** the recesses (3) extend over an angle of less than 50°, preferably less than 40°.

6. The interference screw according to any one of the preceding claims, **characterized in that** the recesses (3) extend substantially along the entire thread (2) in the axial direction, and/or that the recesses (3) do not extend over the tooth crests (6) of the thread (2).

7. The interference screw according to any one of the preceding claims, **characterized in that** the front face of the flanks of the thread, at least portions thereof, extends at a larger angle relative to a plane perpendicular to the central axis of the screw than the rear face of the flanks, and/or that the front face of the flanks of the thread, at least portions thereof, extend at an angle between 25° and 35° relative to a plane perpendicular to the central axis of the screw, and/or that the rear face of the flanks of the thread, at least portions thereof, extend at an angle between 10° and 20° relative to a plane perpendicular to the central axis of the screw (1).

8. The interference screw according to any one of the preceding claims, **characterized in that** the interference screw (1) is porous, at least in portions thereof, in particular **in that** the porosity increases from the outside inwardly.

9. The interfering screw according to any one of the preceding claims, **characterized in that** the degree of porosity is more than 5 %, preferably more than 10 %, and most preferably more than 20 %, at least in portions thereof, and/or that the degree of porosity is at least 1.5 times greater, preferably at least 2 times greater and most preferably at least 3 times greater in a region of the screw away from the screw's surface compared to a region of the screw near the screw's surface.

10. The interference screw according to any one of the preceding claims, **characterized in that** the interference screw (1) has a coating which dissolves within a predetermined time after placement, or which is removable by inductive heating of the interference screw after placement.

11. A method for producing an interference screw according to any one of the preceding claims, **characterized in that** the interference screw (1) is produced by a die cast process in which the casting die is opened along recesses (3) in portions of the thread turns (4) of the external thread (2).

12. The method for producing an interference screw according to the preceding claim, **characterized in that** the recesses (3), at least portions thereof, extend at an angle between 0.1° and 15°, preferably between 0.3° and 0.7° to the opening direction of the casting die.

## Revendications

1. Vis d'interférence (1) comprenant un filet extérieur (2), conçue en particulier pour fixer un ligament, dans laquelle les pas de filetage du filet extérieur présentent au moins par endroits des évidements (3), **caractérisée en ce que** les évidements (3) sont réalisés sous la forme de méplats au moins de la base de la dent et forment au moins par endroits une surface sécante par rapport à au moins un flanc de dent et à une base de dent du filet (2), dans laquelle la surface sécante passe à l'extérieur des flancs de dent avant d'avoir atteint la tête de dent (6) du filet (2).

2. Vis d'interférence selon la revendication 1, **caractérisée en ce que** les évidements (3) sont ménagés sensiblement les uns derrière les autres dans la direction axiale.

3. Vis d'interférence selon l'une quelconque des revendications précédentes, **caractérisée en ce qu'**au moins la moitié, de préférence la totalité, des pas de filetage (4) présentent un évidement.

4. Vis d'interférence selon l'une quelconque des revendications précédentes, **caractérisée en ce qu'**une tangente d'un cercle, dont le centre se situe sur l'axe médian de la vis d'interférence (1), se situe sensiblement sur l'évidement (3) et/ou **en ce que** l'évidement (3) s'enfonce entre 0,05 et 1 mm dans le corps de vis au point le plus profond sur la base de dent (9) du filet (2).

5. Vis d'interférence selon l'une quelconque des revendications précédentes, **caractérisée en ce que** les évidements (3) s'étendent sur un angle inférieur à 50°, de préférence inférieur à 40°.

6. Vis d'interférence selon l'une quelconque des revendications précédentes, **caractérisée en ce que** les évidements (3) s'étendent dans la direction axiale sensiblement le long de l'ensemble du filet (2) et/ou **en ce que** les évidements (3) ne s'étendent pas sur les têtes de dent (6) du filet (2).

7. Vis d'interférence selon l'une quelconque des revendications précédentes, **caractérisée en ce que** la face avant des flancs du filet forme, avec un plan s'étendant perpendiculairement à l'axe médian de la vis, au moins par endroits un angle plus grand que ce n'est le cas avec la face arrière des flancs et/ou **en ce que** la face avant des flancs du filet forme, avec un plan s'étendant perpendiculairement à l'axe médian de la vis, au moins par endroits un angle compris entre 25° et 35° et/ou **en ce que** la face arrière des flancs du filet forme, avec un plan s'étendant perpendiculairement à l'axe médian de la vis (1), au moins par endroits un angle compris entre 10° et 20°.

8. Vis d'interférence selon l'une quelconque des revendications précédentes, la vis d'interférence (1) étant **caractérisée en ce qu'**elle est au moins en partie poreuse, en particulier **en ce que** la porosité augmente de l'extérieur vers l'intérieur.

9. Vis d'interférence selon l'une quelconque des revendications précédentes, **caractérisée en ce que** le degré de porosité atteint au moins par endroits plus de 5 %, de préférence plus de 10 % et de manière particulièrement préférée plus de 20 % et/ou **en ce que** le degré de porosité dans une zone de la vis éloignée de la surface est au moins 1,5 fois, de préférence au moins 2 fois et de manière particulièrement préférée au moins 3 fois plus élevé que celui d'une zone de la vis proche de la surface.

10. Vis d'interférence selon l'une quelconque des revendications précédentes, la vis d'interférence (1) étant **caractérisée en ce qu'**elle comprend un revêtement qui, après l'insertion, se désagrège au cours d'un laps de temps prédéfini ou qui, après l'insertion, peut être retiré à la suite d'un chauffage par induction de la vis d'interférence.

11. Procédé de fabrication d'une vis d'interférence selon l'une quelconque des revendications précédentes, **caractérisé en ce que** la vis d'interférence (1) est fabriquée au moyen d'un procédé de coulée sous pression, au cours duquel le moule de coulée sous pression est ouvert le long d'évidements (3), situés sur certaines parties, des pas de filetage (4) du filet extérieur (2).

12. Procédé de fabrication d'une vis d'interférence selon la revendication précédente, **caractérisé en ce que** les évidements (3) forment avec la direction d'ouverture du moule de coulée sous pression au moins par endroits un angle compris entre 0,1 et 15°, de préférence entre 0,3 et 0,7°.
